# EUROPEAN PATENT SPECIFICATION

(11) **EP 1 562 562 B1**
(45) Date of publication and mention of the grant of the patent: **06.09.2006**
(21) Application number: 03758031.3
(22) Date of filing: 20.10.2003
(51) Int. Cl.: A61K 8/04, A61Q 1/12

(54) **USE OF CELLULOSE MICROBEADS TO MAKE THE SKIN MATT**
VERWENDUNG VON CELLULOSEPERLEN ZUR MATTIERUNG DER HAUT
UTILISATION DE MICROBILLES DE CELLULOSE POUR MATIFIER LA PEAU

(30) Priority: 12.11.2002 FR 0214115; 27.11.2002 US 429342 P
(43) Date of publication of application: 17.08.2005
(73) Proprietor: L'ORÉAL, 75008 Paris (FR)
(72) Inventor: CASSIN, Guillaume, F-91140 Villebon sur Yvette (FR)
(74) Representative: Renard, Emmanuelle
(86) International application number: PCT/EP2003/011646
(87) International publication number: WO 2004/043329

(56) References cited:
- EP-A- 0 432 279
- WO-A-97/32560
- DATABASE KOSMET [Online] IFSCC; S. ISHIHARA ET AL.: "Novel Colored Complex Cellulose Beads" XP002247784 retrieved from KOSMET Database accession no. 26829 & S. ISHIHARA ET AL.: "Poster Presentation P 221" 22 ND IFSCC INTERNATIONAL CONGRESS, 22 September 2002 (2002-09-22), - 26 September 2002 (2002-09-26) Edinburgh cited in the application

## Description

The present invention relates to the cosmetic use of cellulose microbeads, at least 90% of which, in numerical terms, have a diameter of less than or equal to 15 µm in a composition that is suitable for topical application to the skin, as a matting agent.

The invention also relates to a cosmetic process for treating greasy skin, comprising the topical application to the skin of a composition containing cellulose microbeads in a physiologically acceptable medium, at least 90% of which, in numerical terms, have a diameter of less than or equal to 15 µm.

Shiny skin, which is often associated with a substantial secretion of sebum, is a problem that particularly affects adolescents, but may also be manifested in adulthood, due especially to the effect of an overproduction of androgens. It may also be associated with sweat resulting from physical activity or climatic conditions. However, shiny skin is considered as unattractive, all the more so since it often entails poorer staying power of makeup, which has a tendency to degrade visually in the course of the day.

To absorb the sebum and the excess oil of the composition that is not absorbed by the skin, use is conventionally made of powders of natural or synthetic origin, among which mention may be made especially of fillers such as talc, starch, mica, silica, nylon powders, polyethylene powders, poly-β-alanine and polymethyl (meth)acrylate powders. Fillers of this type have the drawback of giving the skin an unnatural powdery appearance, which may even accentuate skin defects. Furthermore, the compositions containing them are generally dehydrating in the long term and their effect is not very long-lasting.

In addition, it has been proposed by the Applicant to use as matting agents vinylpyrrolidone/1-triacontene copolymers (FR-2 820 972) or melamine-formaldehyde or ureaformaldehyde resin particles (FR-2 792 642), styrene-acrylic resin particles (FR-2 801 215), polytetrafluoroethylene resin particles (FR-2 820 977) or colloidal_dispersions of mineral particles, in particular of silica, which may be prepared via a solgel process (EP-0 682 939).

WO-97/32560 discloses cosmetic compositions for reducing greasiness of skin comprising silica beads. EP- 432 279 discloses powdery cosmetic compositions with good spreadability and adhesion to skin comprising cellulose micro beads.

Although these matting agents have advantageous properties, there is still a need for matting agents that have both good compatibility with the skin - and in particular that are of natural origin - and good sensory properties when applied.

The Applicant has now discovered that the use of cellulose microbeads of given size makes it possible to formulate products for greasy skin that have good matting power, while at the same time affording good physiological compatibility, on account of their natural origin, and good cosmetic properties, in the sense that these products do not dry out the skin.

These cellulose microbeads, and the process for preparing them, have been described in the publication by ISHIHARA S. et al., Novel coloured complex cellulose beads, 22^{nd} Congress of the IFSCC, Edinburgh 2002. It is suggested to use them in cosmetics for the formulation of products with a soft feel and good moisturizing power on account of their water-absorbing capacity. These cellulose microbeads are also advantageous for absorbing oil, which makes it possible to envisage their use for improving the staying power of makeup products.

However, it is not suggested in the said document that these cellulose microbeads can have an instantaneous matting effect, i.e. give the skin a matt appearance immediately on application, and that they may thus be of interest precisely in caring for greasy skin.

Now, the Applicant has demonstrated that certain cellulose microbeads, which are precisely such that at least 90%, in numerical terms, have a diameter of less than or equal to 15 µm, have the abovementioned matting properties.

One subject of the present invention is thus a cosmetic process for treating greasy skin, comprising the topical application to the skin of a composition containing cellulose microbeads in a physiologically acceptable medium, at least 90% of which, in numerical terms, have a diameter of less than or equal to 15 µm.

For the purposes of the present invention, the expression "cosmetic treatment of greasy skin" means the topical application of skincare products, intended not only to instantaneously reduce the shininess of the skin, but also to make it healthy by providing it with various active agents intended to combat its imperfections, such as antibacterial or keratolytic agents.

For the purposes of the present invention, the term "microbeads" means solid or porous particles, i.e. microparticles rather than microcapsules, having a circularity parameter of at least 0.95. The circularity parameter is defined as the ratio of the circumference of a disc having the same area as the particle to the perimeter of the particle. A value of 1 characterizes particles that are perfectly spherical.

The microbeads according to the invention are such that at least 90%, in numerical terms, have a diameter of less than or equal to 15 µm. They preferably have a number-average diameter of not more than 10 µm, preferably between 0.1 and 10 µm and better still between 0.5 and 10 µm.

The size distribution of the cellulose microbeads used according to the invention may be measured using an FPIA 2100 image analysis granulometer from the company Malvern.

These microbeads may be prepared especially as described in the abovementioned Ishihara publication, i.e. according to a process comprising the successive steps of mixing an aqueous solution of sodium polyacrylate and of viscose, of stirring the mixture, of heating to 80°C, of filtering, of acidic hydrolysis and of washing with water. They are moreover commercially available in the form of powder from the company Daito under the commercial reference Cellulo Beads D-5® or, better still, from the company LCW under the trade name Covabeads CLO® .

The invention also relates to the cosmetic use of cellulose microbeads, at least 90% of which, in numerical terms, have a diameter of less than or,equal to 15 µm, in a composition suitable for topical application to the skin, as a matting agent.

For the purposes of the present invention, the term "matting agent" means a raw material capable of instantaneously (immediately on application) reducing the shininess of the skin, i.e. the ratio R of the specular reflection to the diffuse reflection, as measured on a gonioreflectometer as described in Example 2 below.

The composition according to the invention is suitable for topical application to the skin and thus generally comprises a physiologically acceptable medium, i.e. a medium that is compatible with the skin and/or its integuments. Thus, the composition according to the invention preferably has a pH of less than 7 and better still between 5 and 6.

The amount of cellulose microbeads present in the composition may vary within a wide range depending on the desired effect. Its very good sensory characteristics allow it to be formulated in fairly high contents, while at the same time maintaining a soft and highly cosmetic texture for the composition containing them. By way of example, these particles may represent from 0.1% to 25% by weight, preferably from 0.5% to 20% by weight and better still from 5% to 15% by weight relative to the total weight of the composition.

The composition according to the invention may be in any presentation form conventionally used for topical application, and especially in the form of aqueous gels or aqueous or aqueous-alcoholic solutions. By adding a fatty or oily phase, they may'also be in the form of dispersions or emulsions of liquid or semiliquid consistency of the milk type, obtained by dispersing a fatty phase in an aqueous phase (O/W) or conversely (W/O), or of suspensions or emulsions of soft, semi-solid or solid consistency of the cream or gel type, or alternatively multiple emulsions (W/Q/W or O/W/O emulsions), microemulsions, vesicular dispersions of ionic and/or nonionic type, or wax/aqueous phase dispersions. These compositions are prepared according to the usual methods.

According to one preferred embodiment of the invention, the composition is in the form of an oil-in-water emulsion.

In this case, the proportion of the oily phase of the emulsion may range, for example, from 1% to 40% by weight relative to the total weight of the composition. The oils, emulsifiers and co-emulsifiers used in the composition in emulsion form are chosen from those conventionally used in cosmetics or dermatology. The emulsifier and the co-emulsifier are generally present in the composition in a proportion ranging from 0.3% to 30% by weight and preferably from 0.5% to 20% by weight relative to the total weight of the composition.

When it is in the form of an O/W emulsion, the composition according to the invention may contain, as surfactants, at least one compound chosen from: polyol esters of fatty acids with a saturated or unsaturated chain containing, for example, from 8 to 24 carbon atoms and better still from 12 to 22 carbon atoms, and the oxyalkylenated derivatives thereof, i.e. derivatives comprising oxyethylene and/or oxypropylene units, such as glyceryl esters of C₈-C₂₄ fatty acids, and the oxyalkylenated derivatives thereof, sorbitol esters of C₈-C₂₄ fatty acids, and the oxyalkylenated derivatives thereof, sugar (sucrose, glucose or alkylglucose) esters of C₈-C₂₄ fatty acids, and the oxyalkylenated derivatives thereof; polyethylene glycol esters of C₈-C₂₄ fatty acids, and the oxyalkylenated derivatives thereof; polyalkylene glycol ethers of C₈₋C₂₄ fatty alcohols; sugar ethers of C₈-C₂₄ fatty alcohols, and mixtures thereof.

As a variant, the composition according to the invention in emulsion form may contain an ionic amphiphilic polymer and may be free of emulsifier.

The composition according to the invention may also contain the adjuvants that are common in cosmetics and dermatology, such as hydrophilic or lipophilic gelling agents, active agents, preserving agents, solvents, fragrances, fillers, pigments, odour absorbers and dyestuffs. The amounts of these various adjuvants are those that are conventionally used in the fields under consideration, for example from 0.01% to 20% of the total weight of the composition. Depending on their nature, these adjuvants may be introduced into the fatty phase or into the aqueous phase. These adjuvants and the concentrations thereof should be such that they do not harm the advantageous properties of the cellulose microbeads according to the invention.

As active agents, the composition according to the invention will preferably contain at least one active agent chosen from: retinoids and in particular retinol; zinc salts such as zinc gluconate; an extract of Laminaria saccharina; an extract of wild yam; triclosan; phenoxyethanol; octoxyglycerol; octanoylglycine; an extract of clove; caprylyl glycol; azelaic acid; α-hydroxy acids such as lactic acid or glycolic acid; β-hydroxy acids, in particular salicylic acid and its derivatives such as 5-n-octanoylsalicylic acid; ursolic acid; panthenol; niacinamide; octopirox.

Examples of fillers that may be mentioned include polyamide (Nylon) particles and especially those sold under the name Orgasol by the company Atochem; polyethylene powders; microspheres based on acrylic copolymers, such as those made of ethylene glycol dimethacrylate/lauryl methacrylate copolymer sold by the company Dow Corning under the name Polytrap; polymethyl methacrylate microspheres, sold under the name Microsphere M-100 by the company Matsumoto or under the name Covabead LH85 by the company Wackherr; ethylene-acrylate copolymer powders, for instance the products sold under the name Flobeads by the company Sumitomo Seika Chemicals; expanded powders such as hollow microspheres, and especially the microspheres formed from a terpolymer of vinylidene chloride, of acrylonitrile and of methacrylate, sold under the name Expancel by the company Kemanord Plast under the references 551 DE 12 (particle size of about 12 µm and density of 40 kg/m³), 551 DE 20 (particle size of about 30 µm and density of 65 kg/m³), 551 DE 50 (particle size of about 40 µm), or the microspheres sold under the name Micropearl F 80 ED by the company Matsumoto; powders of natural organic materials such as starch powders, especially of crosslinked or non-crosslinked maize, wheat or rice starch, such as the starch powders crosslinked with octenylsuccinic anhydride, sold under the name Dry-Flo by the company National Starch; polyamide fibres; silicone resin microbeads such as those sold under the name Tospearl by the company Toshiba Silicone, especially Tospearl 240; silica; metal oxides such as titanium dioxide, zinc oxide or alumina; mica; talc; sericite; boron nitride; clays; and mixtures thereof.

The invention will now be illustrated by the non-limiting examples that follow. In these examples, the amounts are indicated as weight percentages.

### EXAMPLES

### Example 1: Matting cream (O/W emulsion)

### Oily phase:

- Cyclohexadimethylsiloxane 10.00%
- Stearyl alcohol 1.00%
- Glyceryl stearate and polyethylene glycol stearate (100 EO) 2.00%
- Dimyristyl tartrate/cetearyl alcohol/oxyethylenated (25 EO) oxypropylenated (25 PO) lauryl alcohol/oxyethylenated (7 EO) C₁₂₋₁₅ alcohols (Cosmacol PSE from the company Enichem) 1.50%

### Aqueous phase:

- Xanthan gum 0.20%
- Crosslinked poly(2-acrylamido-2-methylpropanesulphonic acid) sold by the company Hoechst under the name Hostacerin AMPS 0.40%
- Sodium hydroxide 0.01%
- Glycerol 5.00%
- Modified starch sold by the company National Starch under the name Dry-Flo 3.00%
- Cellulose microbeads sold by the company Daito under the name Cellulo Beads D-5 3.00%
- Preserving agents 0.70%
- Water qs 100.00%

### Procedure:

The above composition was prepared in the following manner. The aqueous and oily phases were prepared separately by mixing their constituents and then heating to 75°C. The oily phase was then introduced slowly into the aqueous phase with stirring using a Moritz blender at a speed of 4000 rpm for 20 minutes.

### Example 2: Demonstration of the matting effect

### Protocol:

Three compositions A to C were tested, whereby:
- composition A corresponded to that of Example 1,
- composition B was identical to that of Example 1, except that the cellulose microbeads were those sold by the company Daito under the name Cellulo Beads D-10,
- composition C was identical to that of Example 1, except that the cellulose microbeads were replaced with the same amount of silica microbeads sold by the company Miyoshi under the name SB150 (number-average diameter: 3-15 µm).

The Cellulo Beads D-5' cellulose microbeads have a number-average diameter of 10 µm, and are such that 90%, in numerical terms, of the particles have a diameter of less than 15 µm, 50%, in numerical terms, of the particles having a diameter of less than 9 µm. The Cellulo Beads D-10 cellulose microbeads have a number-average diameter of 13 µm, and are such that 90%, in numerical terms, of the particles have a diameter of less than 23 µm, 50%, in numerical terms, of the particles having a diameter of less than 14 µm.

The principle of the method consists in measuring, using a gonioreflectometer, the specular reflection and the diffuse reflection of a deposit of each composition to be evaluated, spread beforehand at room temperature onto a rubber support at a rate of 2 mg/cm². The deposits are then dried at room temperature for 30 minutes.

The matting effect is then evaluated from the ratio R of the specular reflection (measured at an angle of 30°) and diffuse reflection (measured at an angle of 0°).

The lower this ratio, the higher the matting effect. In general, a ratio R of greater than 2 is considered as reflecting an absence of improvement of the appearance of the skin.

### Results:

The results obtained for the three test compositions are collated in the following table:

| **Composition** | **R** |
|---|---|
| A | 1.77 ± 0.04 |
| B | 2.32 ± 0.17 |
| C | 1.91 ± 0.09 |

It is thus seen from the above table that the cellulose microbeads in accordance with the invention have a higher matting power than the cellulose microbeads of larger diameter and than the silica microbeads. It is also seen that composition B containing cellulose microbeads, at least 90% of which have a diameter of less than 15 µm, will have no effect on the matt appearance and the uniformity of the complexion.

## Claims

1. Cosmetic process for treating greasy skin, comprising the topical application to the skin of a composition containing cellulose microbeads in a physiologically acceptable medium, at least 90% of which, in numerical terms, have a diameter of less than or equal to 15 µm.

2. Process according to Claim 1, **characterized in that** the cellulose microbeads have a number-average diameter of between 0.5 and 10 µm.

3. Process according to Claim 1 or 2, **characterized in that** the cellulose microbeads have a circularity parameter of at least 0.95.

4. Process according to any one of Claims 1 to 3, **characterized in that** the cellulose microbeads are obtainable according to a process comprising the successive steps of mixing an aqueous,solution of sodium polyacrylate and of viscose, of stirring the mixture, of heating to 80°C, of filtering, of acidic hydrolysis and of washing with water.

5. Process according to any one of Claims 1 to 4, **characterized in that** the composition contains from 5% to 15% by weight of cellulose microbeads.

6. Process according to any one of Claims 1 to 5, **characterized in that** the composition is in the form of an oil-in-water emulsion.

7. Cosmetic use of cellulose microbeads, at least 90% of which, in numerical terms, have a diameter of less than or equal to 15 µm, in a composition that is suitable for topical application to the skin, as a matting agent.

## Patentansprüche

1. Kosmetisches Verfahren zur Behandlung von fettiger Haut, das das topische Aufbringen einer Zusammensetzung auf die Haut umfasst, die Cellulose-Mikroperlen in einem physiologisch akzeptablen Medium enthält, von denen auf die Anzahl bezogen mindestens 90 % einen Durchmesser von kleiner oder gleich 15 µm haben.

2. Verfahren nach Anspruch 1, **dadurch gekennzeichnet, dass** die Cellulose-Mikroperlen einen zahlenmittleren Durchmesser im Bereich von 0,5 bis 10 µm aufweisen.

3. Verfahren nach Anspruch 1 oder 2, **dadurch gekennzeichnet, dass** die Cellulose-Mikroperlen einen Rundheitsparameter von mindestens 0,95 aufweisen.

4. Verfahren nach einem der Ansprüche 1 bis 3, **dadurch gekennzeichnet, dass** die Cellulose-Mikroperlen nach einem Verfahren erhältlich sind, das die folgenden aufeinander folgenden Schritte umfasst: Mischen einer wässerigen Lösung aus Natriumpolyacrylat und Viskose, Rühren des Gemisches, Erhitzen auf 80 °C, Filtern, saure Hydrolyse und Waschen mit Wasser.

5. Verfahren nach einem der Ansprüche 1 bis 4, **dadurch gekennzeichnet, dass** die Zusammensetzung 5 bis 15 Gew.-% Cellulose-Mikroperlen enthält.

6. Verfahren nach einem der Ansprüche 1 bis 5, **dadurch gekennzeichnet, dass** die Zusammensetzung in Form einer Öl-in-Wasser-Emulsion vorliegt.

7. Kosmetische Verwendung von Cellulose-Mikroperlen als mattierender Bestandteil, von denen auf die Anzahl bezogen mindestens 90 % einen Durchmesser von kleiner oder gleich 15 µm aufweisen, in einer Zusammensetzung, die zum topischen Auftragen auf die Haut geeignet ist.

## Revendications

1. Procédé de traitement cosmétique des peaux grasses, comprenant l'application topique sur la peau d'une composition renfermant, dans un milieu physiologiquement acceptable, des microbilles de cellulose dont au moins 90% en nombre ont un diamètre inférieur ou égal à 15 µm.

2. Procédé selon la revendication 1, **caractérisé en ce que** les microbilles de cellulose ont un diamètre moyen en nombre compris entre 0,5 et 10 µm.

3. Procédé selon la revendication 1 ou 2, **caractérisé en ce que** les microbilles de cellulose ont un paramètre de circularité d'au moins 0,95.

4. Procédé selon l'une quelconque des revendications 1 à 3, **caractérisé en ce que** les microbilles de cellulose sont obtenues selon un procédé comprenant les étapes successives de mélange d'une solution aqueuse de polyacrylate de sodium et de viscose, d'agitation du mélange, de chauffage à 80°C, de filtration, d'hydrolyse acide et de lavage à l'eau.

5. Procédé selon l'une quelconque des revendications 1 à 4, **caractérisé en ce que** la composition renferme de 5 à 15% en poids de microbilles de cellulose.

6. Procédé selon l'une quelconque des revendications 1 à 5, **caractérisé en ce que** la composition est sous forme d'émulsion huile-dans-eau.

7. Utilisation cosmétique de microbilles de cellulose dont au moins 90% en nombre ont un diamètre inférieur ou égal à 15 µm, dans une composition adaptée à une application topique sur la peau, en tant qu'agent matifiant.
